# EUROPEAN PATENT SPECIFICATION

(11) **EP 1 804 867 B1**
(45) Date of publication and mention of the grant of the patent: **10.03.2010**
(21) Application number: 05757555.7
(22) Date of filing: 07.06.2005
(51) Int. Cl.: A61M 5/32

(54) **SHARPS CONTAINER FOR "NO-TOUCH," SEQUENTIAL SAFE STORAGE OF USED PEN NEEDLES**
BEHÄLTER FÜR SPITZE GEGENSTÄNDE FÜR DIE BERÜHRUNGSLOSE SEQUENTIELLE SICHERE AUFBEWAHRUNG VON GEBRAUCHTEN PEN-NADELN
CONTENEUR A POINTES POUR STOCKAGE SUR ET SEQUENTIEL SANS CONTACT D'AIGUILLES USAGEES DE STYLO A AIGUILLE

(30) Priority: 07.06.2004 US 862835; 07.06.2004 US 862622; 07.06.2004 US 862621
(43) Date of publication of application: 11.07.2007
(73) Proprietor: Ultimed, Inc., St. Paul, MN 55101 (US)
(72) Inventor: ERICKSON, THOMAS, E., Crosslake, Minnesota 56442 (US); ERICKSON, JAMES, J., Mound, Minnesota 55364 (US); BACHMAN, Timothy, A., ST. Paul, Minnesota 55116 (US)
(74) Representative: Curley, Donnacha John
(86) International application number: PCT/US2005/020008
(87) International publication number: WO 2005/120611

(56) References cited:
- DE-A1- 3 506 218
- GB-A- 2 376 892
- US-A- 5 152 394
- US-A- 5 573 113
- US-A1- 2003 040 715
- US-A1- 2003 132 129

## Description

### BACKGROUND AND FIELD OF THE INVENTION

This invention relates generally to a "sharps" container for both used pen needles (sometimes hereafter referred to as "PNs") and used medical syringes (sometimes hereafter referred to as "MSs") and specifically to a sharps container for used PNs which provides the safe (no-touch) sequential feeding or insertion of used PNs into the container for safe storage therein. Pen needles are initially received by the user in the form of a pen needle assembly ("PNA"). The invention, importantly, also provides for safe, convenient transportation of a plurality of unused pen needle assemblies (PNAs) within the same sharps container (but hygienically isolated from the used PNs) and for dispensing said unused PNAs from the sharps container.

Because of well known health issues, the safe disposal of "sharps" such as used pen needles, pen needle assemblies, and syringes has long been a high priority for medical related professional facilities and industries. Prior art sharps containers are found in public venues such as hospitals, medical clinics, and retail establishments. These containers are usually securely attached to some base means and have a lock means to permit controlled and safe removal of used "sharps."

There are also prior art "portable" sharps containers for syringes, examples being U.S. Patents 5,494,158 and 6,685,017 showing sharps containers which necessarily are large because of the size of the elongated syringes.

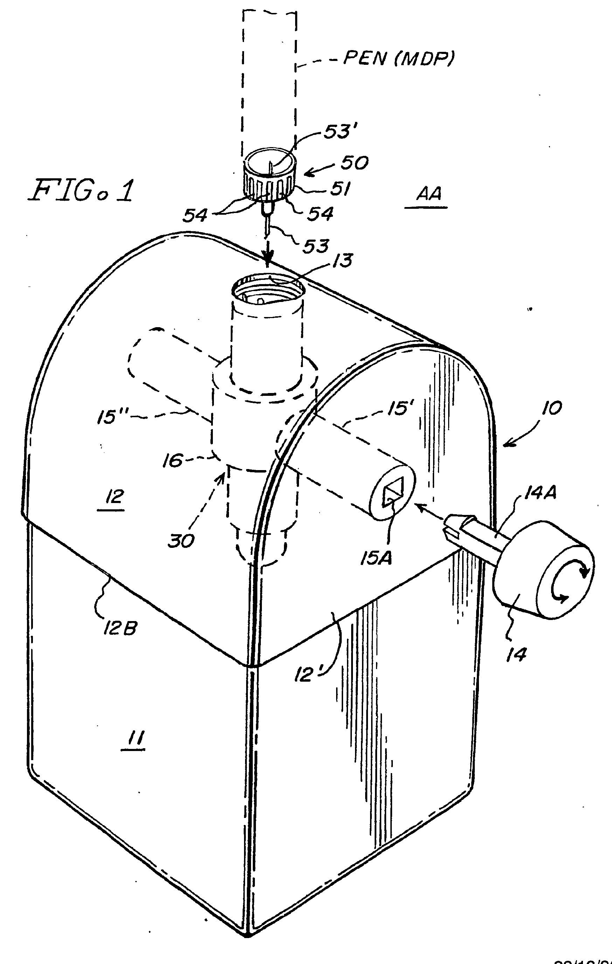

Medical delivery pens (hereinafter sometimes "MDPs") have, more recently, become widely used instead of or in addition to syringes, e.g., by diabetics, who frequently inject themselves several times a day with accurately measured, adjustable, pre-selected amounts of insulin or other medication. Medical delivery pens include a reservoir of medication and a distal end adapted to be attached (usually by thread means) to a pen needle assembly (PNA). As is well known (see, for example Figure 1 of U.S. Patent 5,545,145), the pen needle assembly has (within an outer, generally cylindrical shield 28) a generally cylindrical housing 26 within which is mounted an axially extending hollow needle 21, (i) the proximal end 24 of which punctures a seal in the distal end 16 of the medical delivery pen 10 (to allow the flow there-through of medication) when the delivery pen is screwed into the proximal end of the pen needle cylindrical housing 26, and (ii) the distal end 22 of which is for insertion into tissue of the person requiring the medication. The pen needle assemblies typically include a removable thin sterile seal covering the proximal (large diameter) end of the said outer shield and a removable tube-like shield covering the distal portion of the hollow needle. The assembled pen needle assembly is then factory sterilized. The user of a pen needle assembly removes the seal from the outer shield, screws the pen into the proximal end of the pen needle housing, removes the outer and tube-like shields, sets the medical delivery pen for the desired dose of medication, and then inserts the distal end of the pen needle into the target tissue following which the medical delivery pen is actuated to deliver the desired dose of medication through the hollow needle into said tissue.

Many diabetics routinely administer medication to themselves several times a day by injection of a pre-selected quantity of insulin (or substitute medication) in liquid form; the correct amount of medication can be determined from prior professional medical instruction or by use of convenient portable blood analysis kits which are small, compact and provide rapid indicators of the user's blood sugar level. The several daily injections are often done away from the diabetic's home or residence which has made the use of the portable, convenient medical delivery pens widespread. The aforesaid testing kits and the medical delivery pens are relatively small in size and can easily fit within a woman's purse or equivalent. A typical scenario for a diabetic at a restaurant for a meal is to first use the blood sugar testing kit to obtain an indicator of his or her blood sugar level. This information then facilitates programming or adjusting the medical delivery pen to deliver the desired quantity of medication. Then the pen with an attached PN (a PNA sans the outer protective shield) is used to inject the medication. These steps require a relatively short length of time and can be done with minimum loss of privacy.

Some people requiring multiple daily medicine injections use both medical syringes and medical delivery pens (MDPs) with PNs. For example, a diabetic may use a medical syringe (with a pre-selected amount of medication) at the beginning of the day and then shift to MDPs for subsequent injections that day because of convenience for use outside of their residence and also some users feel less discomfort from a PN injection as compared to that from a syringe needle-type injection.

MDPs are also widely used by doctors, nurses and other professionals in their duties. Many individuals will request (sometimes insist) that an injection be done with a pen needle rather than a syringe. The aforementioned professionals are especially mindful of possible dangers from a needle stick and the possible unwanted "sticks" that occur in the professional world.

In a perfect world, the user (both individual and professional) of a pen needle assembly would, after the first use of a pen needle, carefully detach the used PN from the medical delivery pen and safely dispose said PN. The approved disposal procedure is (i) insertion of the distal end of the needle into the tube-like shield (sometimes omitted) and thence the shielded needle and PN cylindrical housing into the outer shield, (ii) unscrewing of the medical delivery pen from the proximal end of the pen needle cylindrical housing, and (iii) careful placement of the used pen needle assembly into a safe sharps container. Further, in the "perfect" world, the user of a medical syringe would safely dispose the used syringe into a safe sharps container. Alas, the recommended procedure is not always followed. Used (and potentially dangerous) PNs or PNAs are routinely left in unsafe places where third parties may unwittingly be "stuck." Examples of such unsafe places are purses, the pockets on the back of aircraft seats, private and public wastebaskets, garbage cans, dumpsters and empty milk or other unsafe containers.

Further, the above described disposal procedure requires that the user (or associate) handle or hold the PN while the pen is unscrewed therefrom; this creates the possibility of a potentially dangerous "stick". Also, if the user (or associate) tries to insert the PN into the outer shield to form a PNA, then additional handling is again required with the possibility of a "stick"

One prior art example of a container for unused and used pen needle assemblies is U.S. 5,545,145 which shows a tube containing a small number of unused pen needle assemblies arranged in axial alignment. This patent also teaches that, as unused assemblies are removed from one end of the tube, then a used assembly may be inserted into the tube from the other end. The tube is adapted to be attached to the side of a medical delivery pen. This arrangement has significant shortcomings. The capacity is quite limited and, potentially
dangerous "sticks" could occur when a user (or associate) tries to insert a used PN (with or without the protective outer shield) into the used end of the tube.
GB 2376892 discloses an apparatus according to the preamble of claim 1, and for the removal of a needle assembly having a needle hub which is screw-threadedly engaged with a syringe, said apparatus comprising a housing having an opening for insertion of the needle assembly and adjacent portion of the syringe, a socket mounted within the housing in alignment with the opening with a cavity shaped to receive the hub and a coaxial through-bore to accommodate the needle. The socket holds the hub rotationally fast allowing the syringe and needle assembly to be unscrewed, and an ejector means located in the housing ejects the unscrewed needle assembly from the socket into a collection container located within the housing.

### SUMMARY OF THE INVENTION

The present invention provides a sharps container in accordance with the claims which follow.

### BRIEF DESCRIPTION OF THE DRAWINGS

Figure 1 is a top, side isometric view of the preferred embodiment of a PN sharps container provided by the invention.

Figure 2 is a side view, partly in section of the container of Figure 1.

Figure 3 is an enlarged view of that part of the top 12A of the housing that includes a port or opening 13 as shown in Figure 2 and as viewed along section lines 3-3 thereof.

Figure 4 is an enlarged view af the ejector means 30 shown in Figure 2 as viewed along section lines 4-4 thereof and including a pen needle 50 positioned in the ejector means 30.

Figure 5A is a cross-section view of the ejector means 30 as viewed along section lines 5A-5A of Figure 4, this view showing the pen needle 50.

Figure 5B is a cross-section view of the ejector means 30 as viewed along section lines 5B-5B of Figure 4 but with the pen needle 50 removed to enable the showing of ribs 35 of the ejector means 30.

Figures 6 and 7 are somewhat schematic views of the housing and the ejector means showing the ejector means in two orientations when rotated about the axis by the manually rotatable means.

Figure 8 is a cross-section view of the rotatable means as viewed along section lines 8-8 of Figure 2.

Figure 9 is a top, side isometric view of the preferred embodiment of the sharps container provided by our invention; this view shows the rotatable means 220 oriented to receive a used PN 250.

Figure 10 is an isometric view of the same container shown in Figure 9 with the rotatable means 220 oriented to receive a used medical syringe 260.

Figure 11 is a cross-sectional view of the rotatable means.

Figure 12 is a cross-sectional view of the rotatable means as viewed along section lines 4-4 of Figure 11; this view shows a used pen needle 250 positioned in the pen needle ejector assembly.

Figure 12A is a view very similar to Figure 12 except showing the rotatable means rotated clockwise so that cam means within the housing has actuated cam follower means of the ejector member to eject a PN into the container.

Figure 13 is an isometric view of the rotatable means.

Figure 14 is a cross-sectional view of the pen needle ejector assembly (with the elongated ejector member axially displaced to the left of the elongated means) as viewed along section lines 6-6 of Figure 12.

Figure 15 is a top, side isometric view of a preferred embodiment of a PN sharps container provided by the invention.

Figure 16A is a view of a plurality of PNAs connected to a flexible tape and coiled in an unused PNA storage compartment in the container of Figure 15 as viewed along section lines 2-2 thereof.

Figure 16B is a view similar to that of Figure 16A (but for an alternate preferred embodiment of the invention) with a plurality of unused PNAs attached to a tape means but oriented 90 degrees from the orientation of the PNAs in Figure 16A.

Figure 16C is a cross-sectional view of Figure 16B as viewed along section lines 2C-2C thereof.

Figure 17 is a plan view of the tape means 359 used with the PNAs depicted in Figure 16A.

Figure 18 is a plan view of the tape means 369 used with the PNAs depicted in Figure 16B.

Figures 19, 20, and 21 show, respectively, our invention with alternate means in the bottom portion of the housing means for the storage and dispensing of unused PNAs.

Figure 19A is a cross-sectional view of a bin means for dispensing unused PNAs as viewed along section lines 5A-5A of Figure 19.

Figure 20A is a cross-sectional view of a drawer means 380 for dispensing unused PNAs as viewed along section lines 6A-6A of Figure 20.

Figure 21A is a cross-sectional view of the unused PNA storage and dispensing means of the sharps container AA-3.

### DETAILED DESCRIPTION OF THE INVENTION

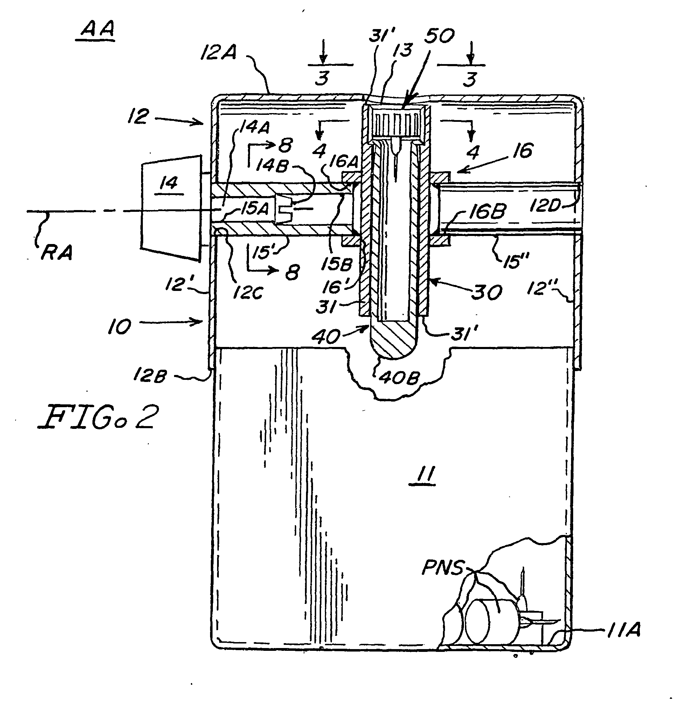

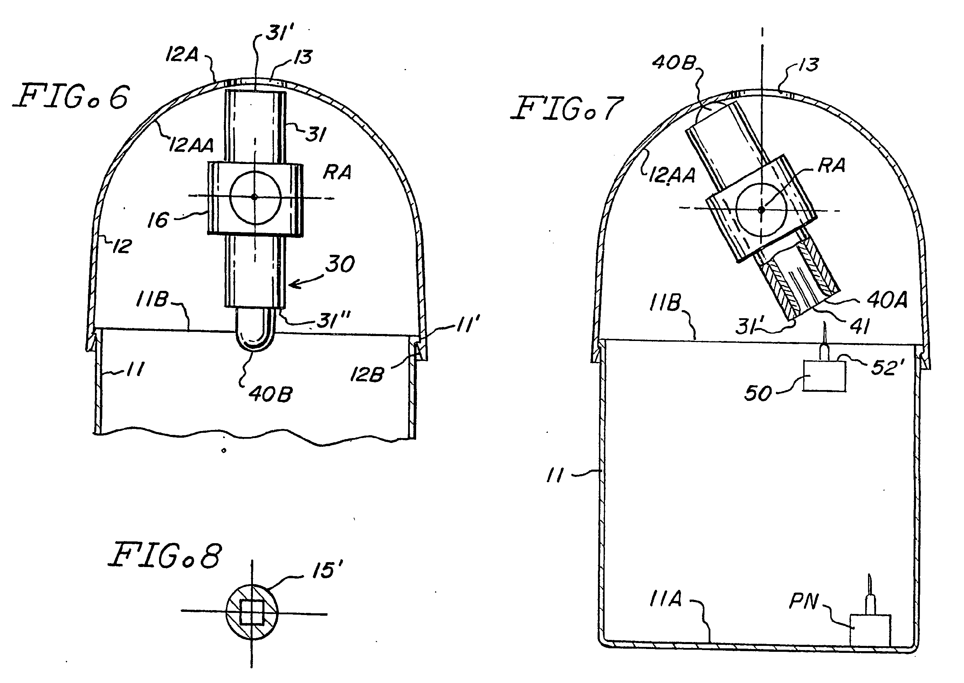

In Figure 1 an illustrative sharps container AA may provide a means for the safe, i.e., "no direct human touching" storage of used pen needles. The container AA may comprise a housing 10 which may have a bottom or storage section 11 and an upper or cover section 12 which may fit together as is shown clearly in Figures 2, 6 and 7 to define an internal storage space sized to facilitate the safe storage of a plurality of used pen needles. Thus section 11 may have sides and a bottom surface 11A for receiving the used pen needles and a top edge 11B with a rim 11'. Cover section 12, as viewed in Figures 6 and 7 may have a curved shape about an axis. The top 12A of the cover section 12 may have an opening 13 sized to permit the axial insertion therethrough of a used pen needle PN also identified in the drawings by reference numeral 50. The bottom edge 12B of the cover section 12 may have an inwardly extending latch portion which co-acts with rim 11' to provide a locking means for sections 11 and 12. Section 12 may have two planar ends 12' and 12" shown best in Figure 2.

Figure 1 shows, in phantom, a medical delivery pen (MDP) of the well known types currently used having at the distal end thereof male thread means for attachment to female threads in the proximal end of a pen needle 50. It should be understood that pen needle 50 shown in Figure 1 may have already been used and the user may desire to safely remove the used pen needle from the pen and thence place the used pen needle into safe storage means. The pen needle 50 may have a cylindrical surface 51 with a pre-selected outer diameter. The cylindrical surface 51 may also have a plurality of longitudinally extending shallow grooves 54 (see Figures 1, 2 and 4) which co-act with ribs 35 of the pen needle receiving and ejecting assembly 30, described below, to hold the pen needle against rotation about its longitudinal axis when the user unscrews the pen therefrom.

A rotatable means, which may be manually rotatable, may comprise an external knob 14 with connected shafts 15' and 15" and a central collar 16 rotatably supported by bearing means 12C and 12D in ends 12' and 12" respectively of the housing for rotation, relative to the housing, about a rotational axis RA. The collar 16 may have a central bore sized to receive and firmly hold the ejector assembly 30 and additionally may have means 16A and 16B for attachment thereto of the inboard ends of shafts 15' and 15". Shaft 15' may have a bore 15A with a square cross-section for receiving a square cross-section shaft 14 A connected to external knob 14. Thus rotation of the knob 14 may rotate the ejector assembly 30 about the rotational axis RA.

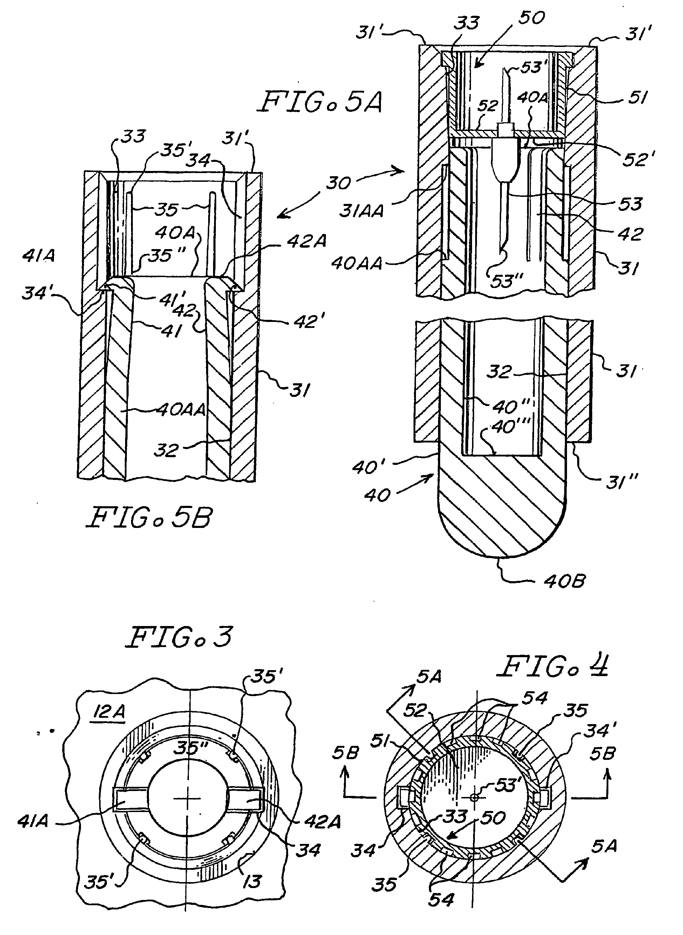

The ejector assembly 30, for this embodiment, is shown to comprise an elongated tubular member 31 having two ends, an upper end 31' and a lower end 31". The upper end 31' per se is best shown in Figure 5B and may comprise a bore 33 sized to receive the aforesaid pre-selected outer cylindrical surface 51 of used pen needle 50. Bore 33 may axially extend from end 31' a pre-selected distance terminating with an inwardly extending shoulder means 34'. A plurality of axially extending ribs 35 may be integral with tubular member 31 and may extend radially inward from the sides of the bore 33 a sufficient amount so as to engage the shallow grooves 54 of the pen needle 50 to provide the above described holding function.

The remaining bore of tubular member 31 may be identified by reference numeral 32; thus bores 32 and 33 together may have a pre-selected axial length, the two ends of which are defined by the ends 31' and 31" of the tubular member 31. Importantly, the ends 31' and 31" may be approximately equidistant from the rotational axis RA. Further, the total axial length of the tubular member 31 may be pre-selected, regard being given to the size of the housing 10, so that it may be rotated about the rotational axis RA without contacting the inside surface 12AA of the housing but yet have the end 31' (for the used pen needle receiving function) adjacent to opening 13 of the housing.

An elongated ejector means 40 may have a pre-selected axial length and a cylindrical shape 40' sized to snuggly, but slidably fit within bores 32 and 33 (for relative axial movement therewith) is shown in Figures 2, 5A, 5B, 6 and 7. At the top of a first end 40A (as shown in Figure 5A) of the ejector means 40, a longitudinally extending bore 40" may be provided which extends to an end surface 40"' which is adjacent to the bottom or second end 40B. End 40B may be curved to provide a cam follower surface. It is important to note that end 40B normally extends a pre-selected distance beyond the end 31" of tubular member 31; this is shown in Figure 5A.

The ejector means 40 may include an integral pair of latch arms 41 and 42 having, respectively, latch means 41' and 42' for engagement with shoulder means 34'; this may provide a holding function to prevent the ejector means 40 from being moved axially out of the tubular member 31 (downward as shown in Figure 5A). However, as described below, the entire ejector means 40 may be, during the used pen needle ejection phase, moved upwardly (as shown in Figure 5A) by the camming action of cam follower 40B contacting the cam surface 12AA on the inside of housing 10. Figure 7 shows the ejector means 40 at its maximum axial displacement relative to the tubular member 31. Such upward, axial motion of the ejector means relative to the member 31, while sufficient to eject the used pen needle, may be limited by a stop means 40AA (on ejector means 40) abutting against an internal shoulder 31AA of member 31.

Figure 5A shows the used pen needle 50 positioned in the recessed bore 33 with the cylindrical surface 51 and associated grooves 54 in firm engagement with bore 33 and its associated ribs 35. Referring to Figure 5B, it may be seen that the ribs 35 may be slightly tapered from top 35' to bottom 35"; this taper function of ribs 35 in combination with reverse tapers of the grooves 54 on the cylindrical surface 51 of the used pen needle may facilitate the aforesaid firm holding of the pen needle. To explain further, when the pen user inserts the pen needle 50 through the opening 13 of the housing and thence into the recessed bore 33, there may be enough axially force applied by the user via the pen to the pen needle to firmly engage the pen needle to the member 31 following which the user may unscrew the pen from the pen needle. Figure 5A also shows the radial wall 52 of the pen needle, and the centrally positioned, axially extending hollow needle 53 having a proximal end 53' and a distal end 53". The bore 40" may be axially sized to accommodate all expected lengths of needles.

Referring to Figure 6, it is seen that the used pen needle receiving and ejecting means 30 may be oriented about the rotational axis RA so that the end 31' may be adjacent to and aligned with the opening 13 of the housing 12. It should be understood, for this explanation, that a used pen needle may already be positioned within bore 33 as aforesaid. In this view, the cam follower 40B is shown extending out end 31' of tubular member 31. To eject the pen needle, the knob 14 may be rotated clockwise as shown in Figure 6; this rotation continues to the orientation shown in Figure 7. Beginning prior to this point the cam follower 40B may have had initial contact with the inside curved surface 12AA of the housing. As the clockwise rotation continues the cam follower (and the entire ejector means 40) may be subject to an axial force tending to move the ejector means 40 in the axial direction toward end 31'. The end result may be that the used pen needle 50may be ejected out of the bore 33 into the bottom of the housing as is shown in Figure 7. Note in Figure 7 that the end 40A of the ejector 40 may be co-planar with end 31' of tubular member 31. The magnitude of the axial force applied to the used pen needle 50 during the ejection may be a function of the level of co-action between the ribs 35 and grooves 54; the force may vary to impart a range of velocities to the ejected pen needle. In all cases, the invention may provide for the safe storage of the used pen needles; the ejection velocity of the used pen needle may be irrelevant because they are confined within the housing.

The apparatus may then be available to safely dispose of additional used pen needle assemblies. The user may rotate the knob 14 to an angular position as shown in Figures 1 and 6 whereat the next used pen needle (on the end of a pen) may be inserted, via opening 13, into the bore 33.

It will be understood that the pen user does not have to touch the used pen needle either to (i) remove the used pen needle from the pen, or (ii) dispose the used pen needle into a safe storage means.

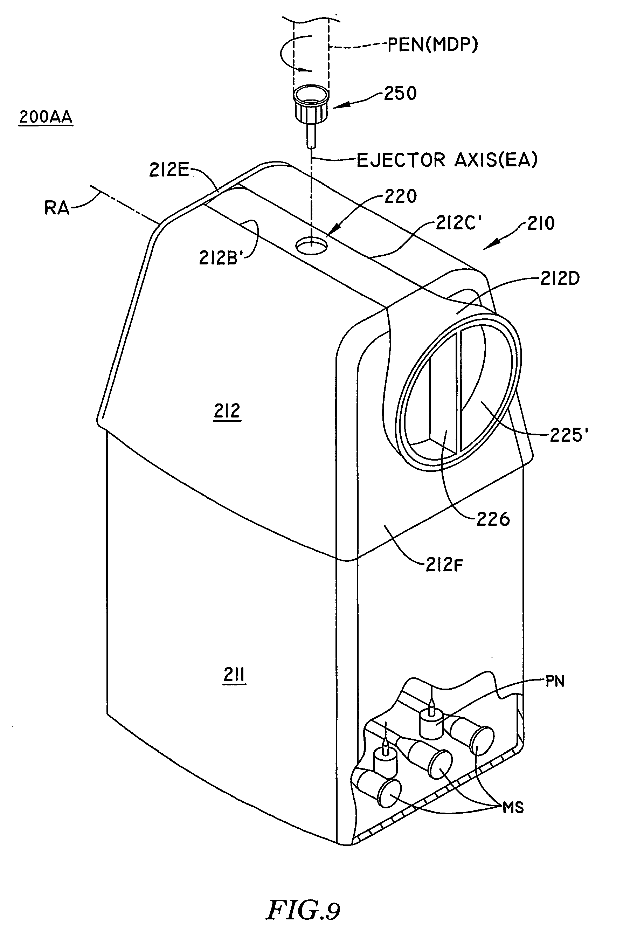

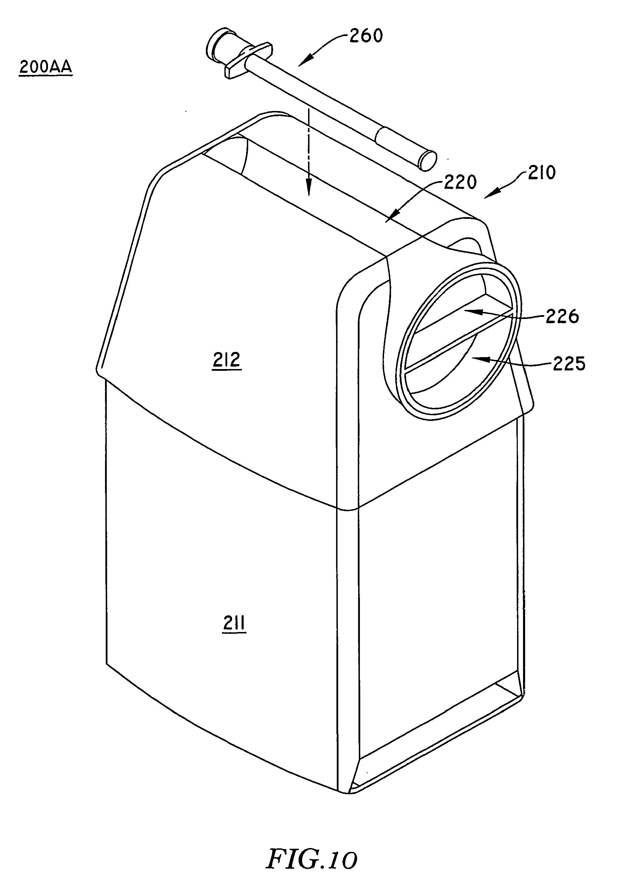

In Figure 9 an illustrative multi-function sharps container 200AA may provide a single apparatus for both the safe, i.e., "no direct human touching" storage of used pen needles and for the safe storage of medical syringes. The container 200AA may comprise a housing 210 which may include a first portion and a second portion, which may be a bottom or storage section 211 and an upper or cover section 212 which may fit, and lock, together as is shown clearly in Figures 9, 10 and 12A to define an internal storage space sized to facilitate the safe storage of a plurality of used pen needles and medical syringes. Thus section 211 may have sides and a bottom surface 211' defining a cup-like means for receiving used pen needles and medical syringes and a top edge with a rim 211A having an outward extending shoulder. Cover section 212 may have a longitudinally extending curved cross sectional shape shown in Figures 9, 10, 12 and 12A. The top of cover section 212 may have a longitudinally extending opening defined by laterally spaced-apart sides 212B' and 212C', the spacing being pre-selected to admit an axially oriented medical syringe 260 as is depicted in Figure 10.

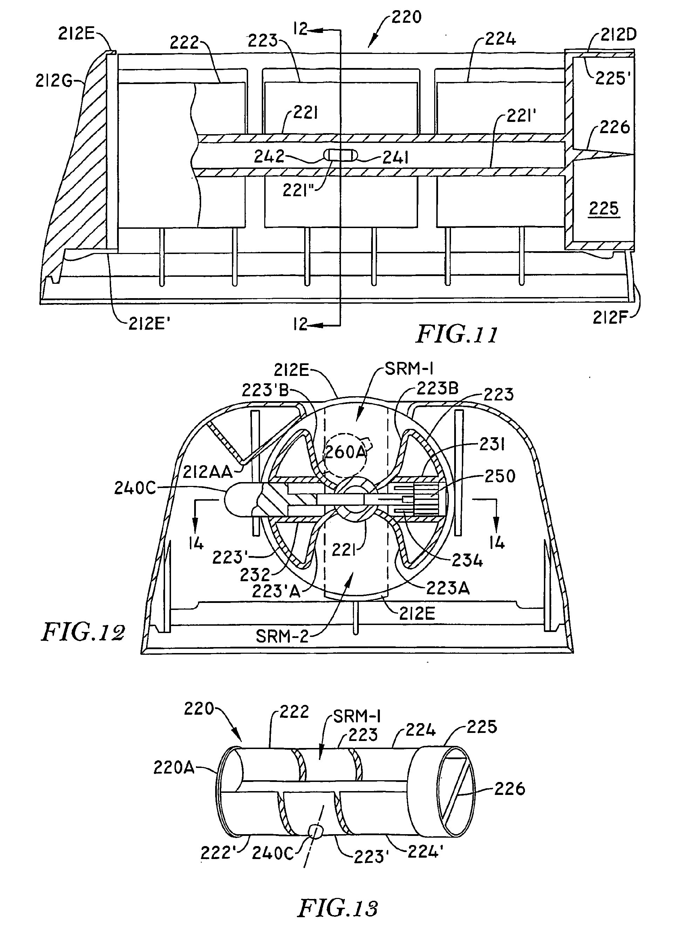

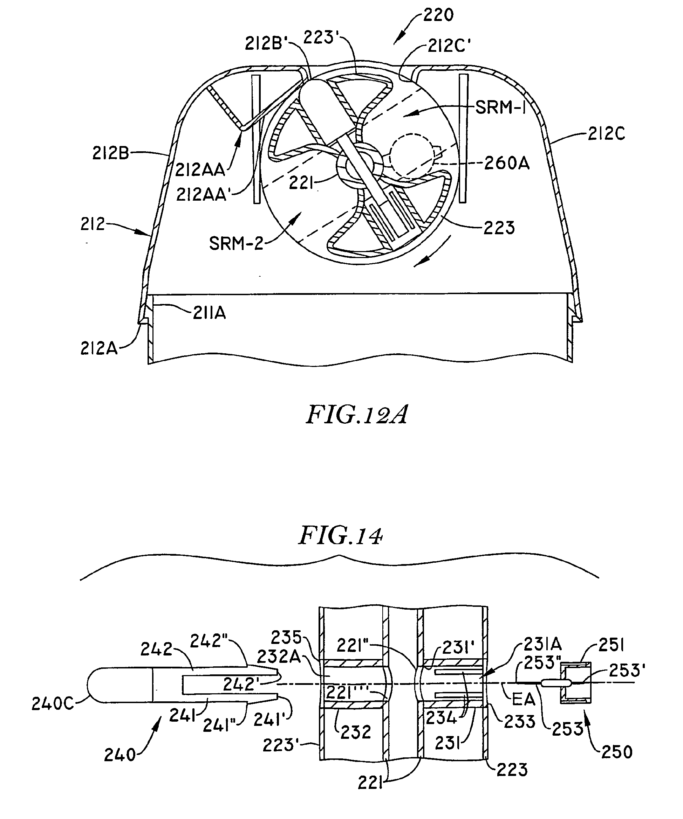

Importantly, Cover 212 may include an internal partition 212AA having a cam surface means 212AA' (see Figures 12 and 12A); the cam surface means 212AA' may be positioned for an initial contact with cam follower means 240C (described below) as the rotatable means 220 is rotated and, after additional rotation thereof, cause the ejection of a PN into the container. Cover 212 may have a lower, inward extending rim 212A adapted to coact with said shoulder on rim 211A of section 211 to lock the sections 211 and 212 together as shown in Figure 12A.

Cover 212 also has opposed end portions 212D/212F and 212E/212E' for rotatably supporting a rotatable means 220 for rotation about a rotational axis RA. The rotatable means 220 may comprise an elongated cylindrically shaped member (see, in particular, Figure 13) having a central hollow core 221 connected at the ends thereof to bearing elements 220A and 225. Bearing element 220A may be supported by cover end portions 212E/212E' and bearing element 225 may be supported by cover end portions 212D/212F. Bearing element 225 may further serve as a means for rotating the rotatable means; it has a circular, longitudinally extending outer rim 225' and a cross rib 226 adapted for engagement by the fingers of a human user for manual rotation of the rotatable means 220.

Integrally connected to the hollow core 221 may be three axially aligned sets of fan shaped segments 222/222', 223/223' and 224/224' (see Figures 11, 12 and 13). The segments may be axially aligned as best shown in Figure 13. Each segment in said set of segments may have exterior curved surfaces concentric with core 221 and extending circumferentially approximately 90 degrees; Figure 12 shows the opposed curved surfaces 223 and 223'. The rotatable means 220 may further include a pair or opposed, longitudinally extending used syringe receiving means SRM-1 and SRM-2 (see Figures 12 and 12A) defined by inwardly extending curved surfaces of segments 222/222', 223/223' and 224/224'; for example Figure 12 identifies curved surfaces (i) 223A and 223A' and (ii) 223B and 223B' for defining the center (of three) portions of SRM-2 and SRM-1 respectively.

The curved exterior surface of segment 223 may have an opening 233 (Figures 9 and 14) for admitting the axial insertion of a PN 250 into a pen needle receiving and holding recess 231A described in more detail below. The curved exterior surface of segment 223' may have an opening 235 sized to admit the relative axial movement of the cam follower end of an ejector member 240. Openings 233 and 235 may define an ejector axis EA (see Figures 9 and 14). The ejector assembly may further comprise, in part, a pair of axially aligned tubular elements 231 and 232 positioned along the ejector axis EA and connected to and radially extending from core 221 on opposite sides thereof as shown in Figures 12 and 14. Core 221 may have a pair of aligned, opposed longitudinally elongated slots 221" and 221'" through which spaced-apart legs 241 and 242 on one end of the ejector member 240 may be inserted to form the assembly shown in Figures 12 and 12A. The ejector member 240 may further have a rounded cam follower surface 240C at the other end thereof. The total axial length of ejector member 240 may be pre-selected so that the axial ends 241' and 242' of legs 241 and 242 may be initially in abutting contact with the radially extending portion of a pen needle positioned in pen needle receiving means 231A in the outboard end of tubular element 231 as is shown in Figure 12; concurrently the cam follower surface 240C may initially extend beyond the surface 223' a pre-selected amount as also is shown in Figure 12. The legs 241 and 242 may have latch means 241" and 242" respectively which, after insertion of the legs through slots 221" and 221"', coact with shoulder means 231' at the inner end of tubular element 231 to hold member 240 in assembled relation with member 220.

Pen needle receiving means 231A may include a plurality of inwardly extending ribs 234 for gripping an inserted PN and preventing rotation of the PN about the ejector axis EA.

Figure 9 shows, in phantom, a medical delivery pen (MDP) of the well known types currently used having at the distal end thereof male thread means for attachment to female threads in the proximal end of a pen needle 250. It should be understood that pen needle 250 shown in Figure 9 may have already been used and the user desires to safely remove the used pen needle from the pen and thence place the used pen needle into safe storage means. The pen needle 250 may have a cylindrical surface 251 with a pre-selected outer diameter. The cylindrical surface 251 may also have a plurality of longitudinally extending shallow grooves 254 which co-act with ribs 234 of the pen needle receiving recess 231A to hold the pen needle against rotation about its longitudinal axis when the user unscrews the MDP therefrom.

To safely dispose and store a used PN, the user may hold the MDP to axially guide the attached PN 250 through opening 233 of the rotatable means 220 into recess 231A of the pen needle receiving and ejecting means, the coacting ribs 234 and shallow slots 254 preventing rotation of the PN about the ejector axis EA to thus facilitate the manual unscrewing of the MDP from the PN. Figure 12 shows a PN in recess 231A, the rotatable means 220 may have been rotated 90 degrees clockwise following the insertion of the PN into the recess. At this orientation the rounded cam follower end 240C may be approaching the housing cam means 212 AA'; continued clockwise rotation of the rotatable means 220 brings end 240C into contact with cam means 212 AA' to initiate axial movement of the ejector member 240 along the ejector axis EA to the right as shown in Figure 12, said axial movement being transferred by the end surfaces 241' and 242' of member 240 to the PN. As the clockwise rotation continues to the position shown in Figure 12A the ejector member 240 may have moved axially sufficiently to eject the PN into the container as is also depicted in Figure 12A.

Additional used pen needles may be sequentially safely disposed and stored by following the same procedure. The rotatable means 220 may be oriented with the opening 233 in the position shown in Figure 9.

To safely dispose and store a medical syringe, the rotatable means 220 may be oriented with one of the syringe receiving means SRM-1 or SRM-2 in the position shown in Figure 12, i.e. with a SRM in register with the cover opening defined by cover edges 212B' and 212C'. Then, as depicted in Figure 10, the used syringe 260 may be placed into the SRM. Next the rotatable means 220 may be rotated manually either direction sufficiently for the used syringe to fall away and down into the storage area of the section 211 of the container. Figure 12A depicts the clockwise rotation of the rotatable means 220 carrying a used syringe 260A positioned in Sum-1; continued clockwise rotation may discharge the syringe into section 211 of the container.

Thus the user of medical syringes and pen needles may have a single container for the safe disposal and storage of both sharps after the use thereof. It will be understood that the pen user does not have to touch the used pen needle either to (i) remove the used pen needle from the pen, or (ii) dispose the used pen needle into a safe storage means. The medical syringe receiving and disposal means provided by our invention may increase the safety for the user.

The syringe receiving means SRM-1 and SRM-2 may, besides the specifically described function of receiving medical syringes (MSs), may also be used to receive used pen needle assemblies (PNAs) for disposal into the container.

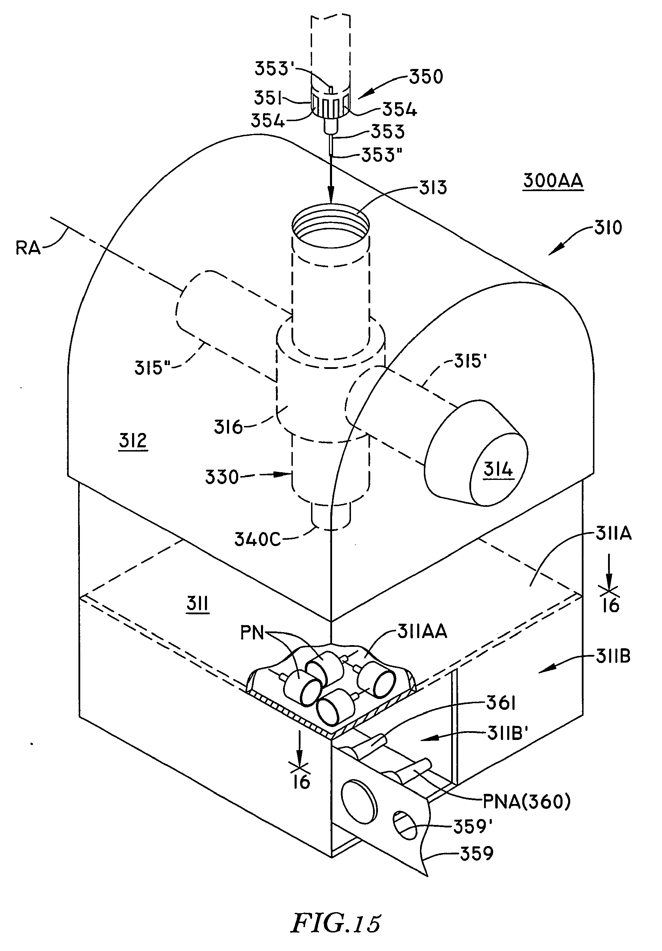

Figure 15 shows an illustrative sharps container 300AA having a means for the safe, i.e., "no direct human touching" storage of used pen needles. The container may comprise a housing 310 having a first or bottom storage and dispensing portion 311 and a second or upper cover portion 312 which fit together as shown. The first portion 311 is, in turn, divided into two parallel oriented portions 311A and 311B by a partition 311AA integral with portion 311 and positioned to facilitate (i) the safe storage of a plurality of used PNs and (ii) the safe storage and dispensing of unused PNAs as is shown in Figure 15.

Cover portion 312 of the housing means 310 may have a curved shape about a rotational axis RA. The top of the cover 312 may have an opening 313 sized to permit the axial insertion therethrough of a used pen needle PN identified in the drawings by reference numeral 350

Figure 15 also shows, in phantom, a medical delivery pen (MDP) which is representative of the well known types currently used and having at the distal end thereof male thread means for attachment to female threads in the proximal end of a pen needle 350. It should be assumed that pen needle 350 shown in Figure 15 may have already been used and the user desires to safely remove the used pen needle from the pen and thence place the used pen needle into safe storage means. The pen needle 350 may have a cylindrical surface 351 with a pre-selected outer diameter. The cylindrical surface may also have a plurality of longitudinally extending shallow grooves 354 which co-act with radially extending ribs of a used pen needle receiving and ejecting assembly 330 to hold the pen needle against rotation about its longitudinal axis when the user unscrews the MDP therefrom. Set forth below is a summary of the details and functions of the used pen needle receiving and ejecting assembly.

A rotatable means may comprise an external knob 314 with connected shafts 315' and 315" and a central collar 316 rotatably supported by bearing means in end walls of the housing cover 312 section for rotation, relative to the housing, about a rotational axis RA. The collar 316 may have a central bore sized to receive and firmly hold the used pen needle receiving and ejector assembly 330 and additionally may have means for attachment thereto of the inboard ends of shafts 315' and 315". Thus rotation of the knob 314 will rotate the ejector assembly 330 about the rotational axis RA.

The ejector assembly 330, for this illustrative embodiment, is shown to comprise a first member or elongated tubular member having first and second ends. The total axial length of the elongated tubular member may be pre-selected, regard being given to the dimensions of the cover section 312, so that the tubular member may be rotated about the rotational axis without contacting the inside surface 312 of the housing but yet have the used pen needle receiving end thereof sufficiently adjacent to the opening 313 to provide the used pen needle receiving function.

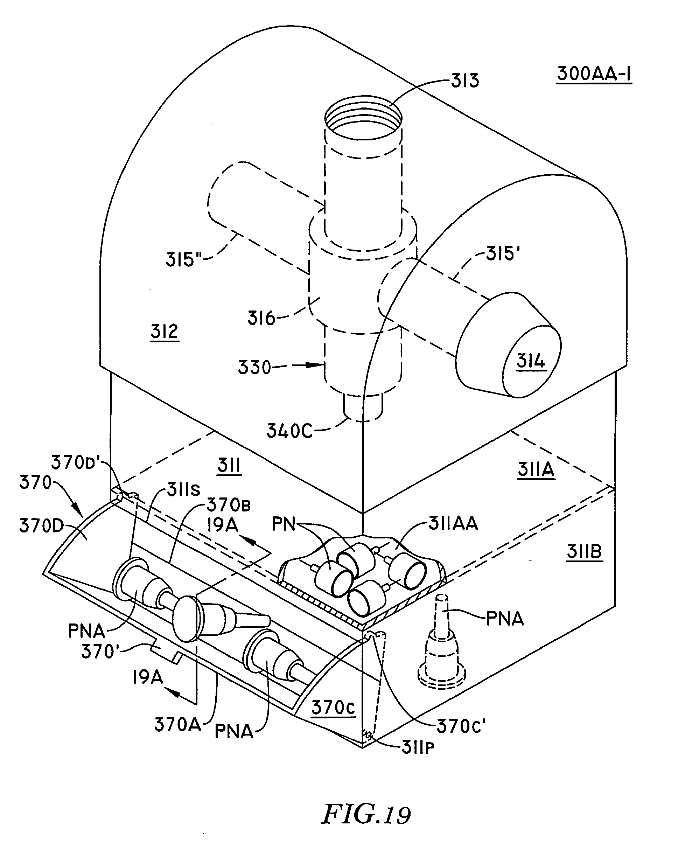

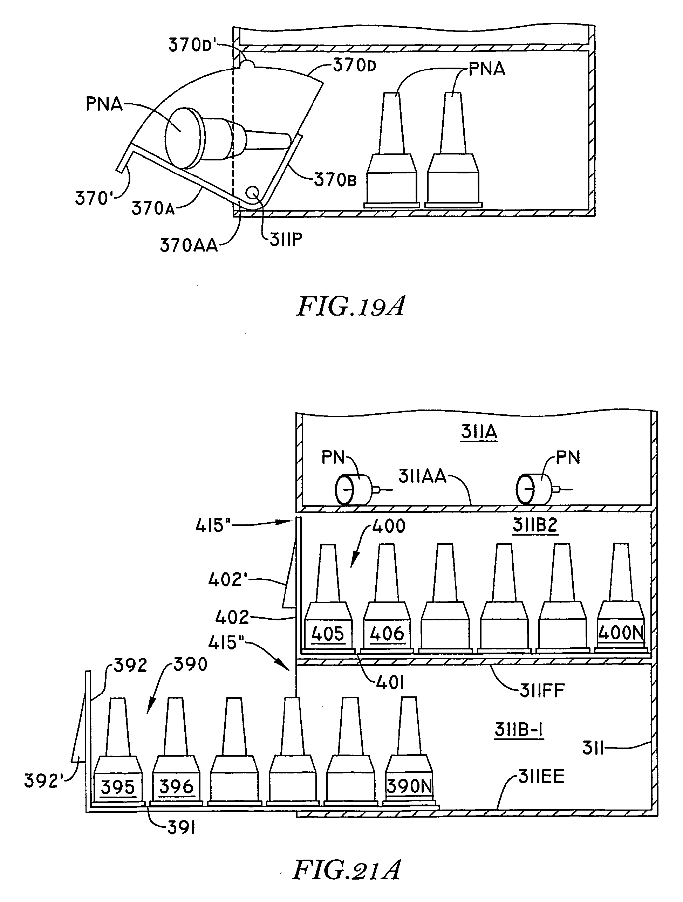

An elongated ejector means may have a pre-selected axial length and a cylindrical shape sized to slidably fit within the elongated tubular member for relative axial movement therewith; a rounded cam follower end 340C of the ejector means being shown in Figure 15. It is important to note that end 340C of the ejector means may normally, i.e. initially, extends a pre-selected distance beyond one end of the tubular member as is shown in Figure 19A. The terms "normally" and "initially" may cover the case when the cam follower end 340C is not in contact with its co-acting cam means. The co-acting cam means may be positioned within and fixed to the cover section 312 and may, in fact, be the inside curved surface of cover section 312.

Thus, rotation of the knob 314 (and thus the entire used pen needle receiving and ejecting means 330) may cause contact by the cam follower 340C with the aforesaid cam means to force the ejector means axially within the elongated tubular means to push, i.e. eject a used pen needle out from its received position into the used pen needle storage portion 311A.

It will be understood that the pen user may not have to touch the used pen needle either to (i) remove the used pen needle from the pen, or (ii) dispose the used pen needle into a safe storage means.

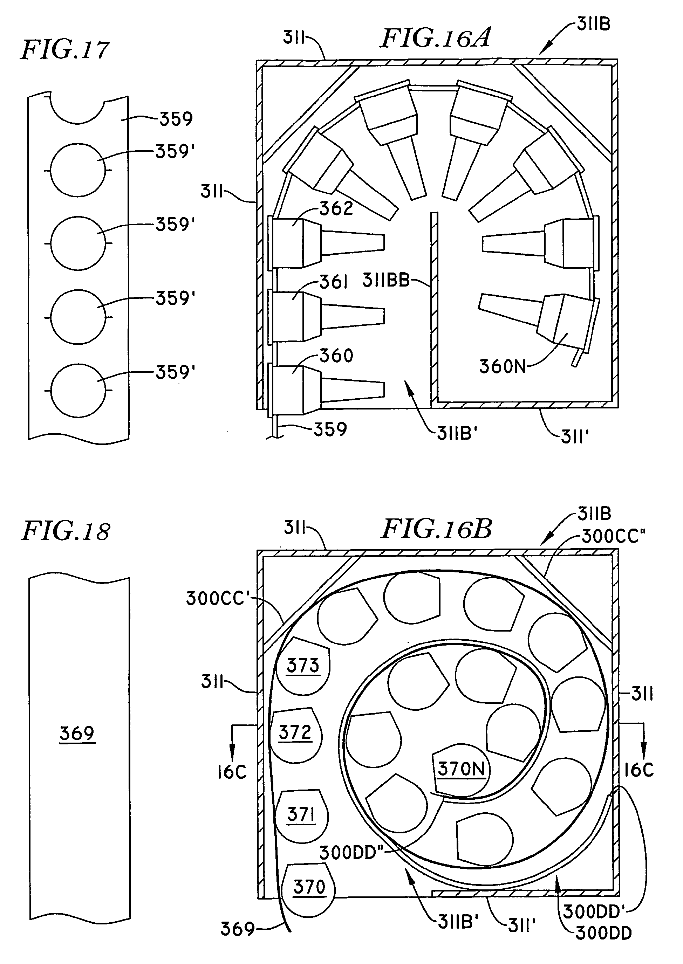

The illustrative unused PNA storage and dispensing portion 311B of the housing is shown in several, alternate configurations. The first is shown in Figure 16A. One of the side walls 311' of storage section 311B may include an exit opening 311B' and an internal side wall 311BB to permit withdrawal of unused PNAs 360, 361, 362......360N attached to a flexible tape means 359 by being positioned in serial, spaced apart holes 359' in the tape as is shown in Figure 17. Figure 16A shows clearly the PNAs within the storage space, coiled around side wall 311BB, and exiting at opening 311B'. Figure 15 also shows the tape 359 with connected PNAs 360 et seq. available, upon demand, to the MDP user at exit opening 311B'; the user of the unused PNAs may pull on the end of the tape 359 to receive the desired number of unused PNAs.

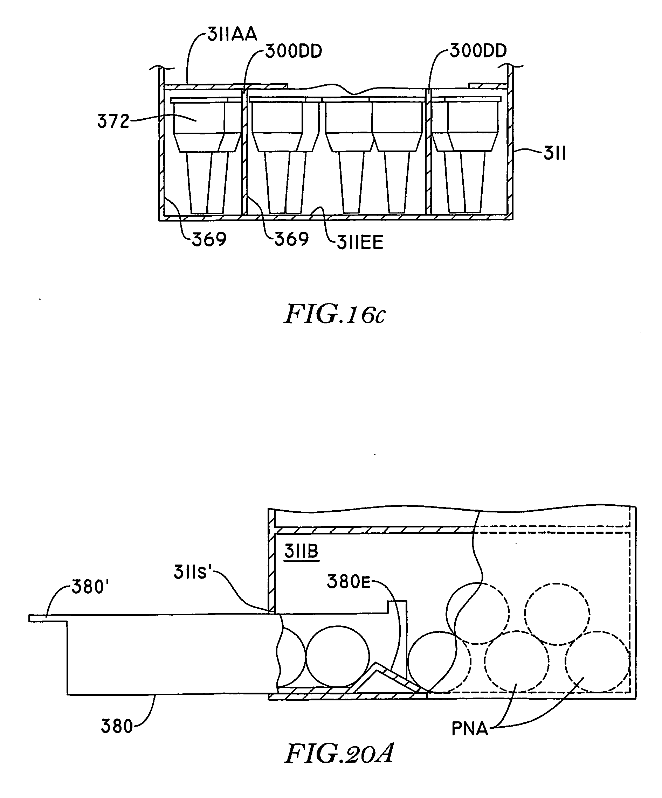

An alternate unused PNA storage and dispensing configuration 311B is shown in Figures 16B and 16C. Again, a side wall 311' may have an exit opening 311B'. Within the storage and dispensing portion 311B may be (i) a pair of angled interior walls 300CC' positioned in the corners as shown and (ii) a spiraled guide means 300DD having a first end 300DD' attached to a side 311 of the portion and a second end 300DD" generally centrally positioned in the portion. A plurality of unused PNAs (370, 371, 372, 373.......370N) may be depicted connected in a serial, spaced-apart configuration to a tape 369. The tape 369 with attached PNAs may be configured in a coil and is shown following the spiral guide means 300DD. The PNAs may be attached to the tape 369 by suitable means such as an adhesive, the cylindrical sides of the PNAs may be the zone of attachment to the tape. The spiral guide means 300DD thus facilitates the loading and dispensing of unused PNAs into and out of the storage space.

Other configurations for the storage and dispensing the unused PNAs also may be used. The configuration depicted in Figures 16B and 16C may have an advantage of an increased number of PNAs being storable in a given container footprint as compared to that of Figure 16A.

It will be noted that the configurations depicted in Figures 15-16C may be especially useful in the sequential dispensing of unused PNAs, the user may pull out only the next available PNA. However, the user may pull out several PNAs if that were desired.

The following embodiments of our invention facilitate the simultaneous safe dispensing of a plurality of unused PNAs from the sharps container; for some applications such as use in a clinic, this function may be very desirable.

Figure 19 shows a sharps container 300AA-1 which may be very similar to container 300AA shown in Figure 15; it may have the same used PN receiving, ejecting and storage means as container 300AA. However, the unused PNA storage and dispensing means may be different; it may be configured to facilitate the dispensing of more than one unused PNA at the same time, i.e., a means to provide unused PNAs in bulk quantities.

Thus container 300AA-1 may have a housing means with partition 311AA separating used PN storage portion 311A from unused PNA storage and dispensing portion 311B. An exit opening 311 S at the left side of portion 311B (as viewed in Figure 19) may be provided, within which may be a bin-like member 370 that may be rotatably positioned. Bin member 370 may have (i) two angularly-displaced-apart longitudinally extending surfaces 370a and 370b, joined at 370AA to form a V-shaped cross section as is shown in Figure 19A and (ii) end surfaces means 370c and 370d to form a unitary pocket means sized to hold a plurality of unused PNAs. Bin member 370 may be supported by bearing means 311P so that it may be rotated (by use of a tab 370') between an open or dispensing position as shown in Figure 19 and a closed position where surface 370a may be coplanar with the side 311 of the housing. When bin member 370 may be in the closed position, additional unused PNAs may be transferred into said pocket as can be seen in Figure 19A. End surface means 370c and 370d have stop means 370c' and 370d' to limit the outward rotation of the bin member 370.

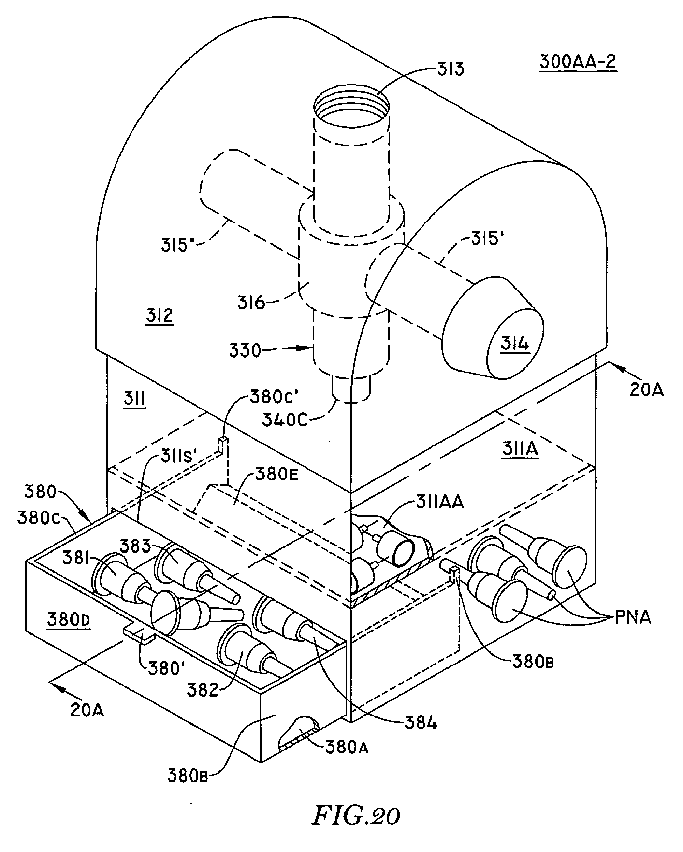

Figure 20 depicts another sharps container 300AA-2 which may have the same used PN receiving, ejecting and storage means as container 300AA of Figure 15 but may provide an alternate, multiple unused PNA dispensing apparatus. Thus container 300AA-2 may have partition 311AA for defining used PN storage portion 311A and unused PNA storage and dispensing portion 311B. An exit opening 311S' at the left side of portion 311B (as viewed in Figure 20) may be provided and may be sized to admit the transverse motion of a drawer 380 in and out of the housing to an unused PNA dispensing position shown in Figure 20. The drawer has a bottom 380a, two sides 380b and 380c, a front 380d (with transversely extending pull tab 380'), and back ramp-like means 380e. Stop means 380c' and 380d' on the sides of the drawer limit the outward travel thereof. In operation, the drawer, holding unused PNAs 381, 382, 383 and 384, may be manually pulled out (by the user using tab 380') from portion 311B to the position shown in Figure 20 to allow the user to remove said unused PNAs. Manual closing of the drawer 380 may result in the front 380d being coplanar with the side 311 of the housing and, importantly, the ramp means 380e, as the drawer is closing, coacts with unused PNAs in portion 311b to transfer some into the drawer for additional dispensing to a user as is clearly shown in Figure 20A.

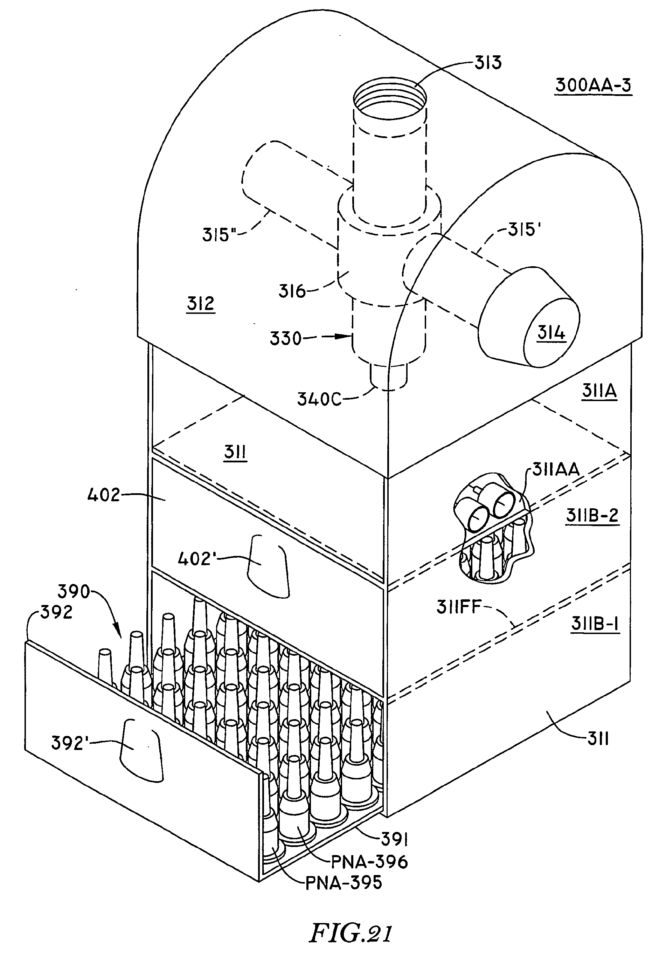

Another embodiment of our invention may be the sharps container 300AA-3 depicted in Figures 21 and 307A which may also use the same used PN receiving, ejecting and storage means as container 300AA. Partition 311AA may again define the used PN storage portion 311A; however the unused PNA portion may be divided into two sub-portions 311B-1 and 311B-2 defined by a partition 311FF integral with side 311 of the housing and extending parallel to the bottom 311EE of the housing (best shown in Figure 21A). The vertical height of the sub-portions may be pre-selected to allow for the storage of unused PNAs arranged with the proximal ends thereof abutting the bottom members 391 and 401 of a pair of tray means 390 and 400 respectively. Tray 390 may also have a front 392 sized to match the exit opening 4415" between members 311EE and 311FF. Tray 400 may also have a front 402 sized to match the exit opening 415"' between members 311A and 311FF. Fronts 392 and 402 may have engagement means 392' and 402' respectively for facilitating the manual withdrawal of the trays from the housing means.

A large number of unused PNAs may be positioned on each of trays 390 and 400. Tray 390 has PNAs 395, 396 ......390N and tray 400 has PNAs 405, 406......400N; the PNAs, as indicated, may be oriented with the proximal ends abutting their respective trays. The PNAs may be secured by a suitable adhesive means to the trays. This orientation of the PNAs may permit an increase in the number of unused PNAs for a given size container.

While we have shown our preferred embodiments of the invention, it will be understood that variations may be made without departing from the inventive concept. Accordingly, the invention is to be limited only by the scope of the following claims.

## Claims

1. A sharps container for storage of used pen needles comprising:
a housing (10) with a container storage space (11) sized to facilitate the storage of a plurality of used pen needles, the container including an opening (13) in said housing sized to admit the axial insertion of a used pen needle therethrough, and used pen needle receiving and ejecting means within said housing, wherein the used pen needle receiving and ejecting means comprises:
a rotatable means (15,16) connected to said housing for rotation relative to said housing about an axis (RA); an ejector assembly (30) connected to said rotatable means to be rotated about said axis; and cam means (212) within said housing wherein the ejector assembly comprises:
an elongated tubular member (31) having recessed means (33) at a first end thereof sized to receive a used pen needle (50);
an ejector member (40', 240) connected to said elongated tubular member for relative axial movement therein along an ejector axis and having
(A) cam follower means (40B, 240C) at one end thereof initially positioned a pre-selected axial distance along said ejector axis beyond a second end of said elongated tubular member, and
(B) a second end initially positioned adjacent said recessed means (33) for receiving the used pen needle; and wherein said cam means is positioned for an initial contacting of said cam follower means when said rotatable member and said ejector assembly is rotated about said rotational axis, wherein said cam means moves said ejector member (240) axially with respect to said elongated tubular member toward said first end so that a second end of said ejector member (40', 240) provides a pen needle ejecting motion whereby, when a pen needle (50) is positioned in said recessed mean (33) and said rotatable means (15,16) is rotated about said axis (RA) sufficiently so that said cam follower means contacts said cam means, said ejector member will force said pen needle axially out of said recessed means into said container storage space (11),
**characterised in that** said ejector assembly (30) is selectively oriented about said axis (RA) by rotation of said rotatable means between
(i) an initial position with said recessed means being adjacent to and in alignment with said opening (13) to facilitate the axial insertion of a used pen needle into said recessed means, and
(ii) a second position whereat said cam follower means engages said cam means, following which additional rotation of said ejector assembly about said axis (RA) causes the ejection of a used pen needle from said recessed means into said housing for safe storage therein.

2. The sharps container of claim 1 wherein said ejector assembly (30) comprising a member having
(i) elongated bore means of pre-selected axial length and first and second ends, said first end of said bore means being sized to receive said pre-selected outer diameter of a pen needle,
(ii) said bore means having shoulder means (231) adjacent to said first end, and
(iii) elongated ejector means positioned within said bore means with latch
means (241,242) at one end thereof for engagement with said shoulder means and with cam follower means at a second end thereof, said ejector means being axially sized so that said cam follower means extends beyond said second end of said bore means when said latch means is in engagement with said shoulder means,
wherein said cam means moves said latch means (241) of said ejector means axially away from said shoulder means toward said first end of said bore means to provide an ejecting motion.

3. The sharps container of claim 2 wherein said ejector assembly comprises a tube means of pre-selected axial length having first and second ends and an internal bore within which is movably positioned said ejector means.

4. The sharps container of claim 3 wherein said internal bore includes a shoulder adjacent to said first end of said tube means and the portion of said internal bore between said first end of said tube means and said shoulder means is sized to closely receive said pre-selected outer diameter of a pen needle.

5. The sharps container of claim 4 wherein said portion of said internal bore includes radially extending means for gripping said pre-selected outer diameter of a pen needle.

6. The sharps container of claim 4 including an opening in said housing sized to allow the axial insertion of a pen needle therethrough, and said used pen needle receiving and ejecting means being oriented with respect to said opening so that said first end of said bore means may be rotated into close proximity to said opening to facilitate the insertion of an used pen needle therein.

7. The sharps container of claim 4 wherein said cam means is integral with said housing.

8. The sharps container of claim 1 wherein said recessed means of said elongated member includes radially extending means.

9. The sharps container of claim 1 further comprising:
used medical syringe receiving and disposal means comprising means included in said rotatable means
(i) for receiving a used medical syringe (260), and
(ii) following a pre-selected rotation of said rotatable means about said rotational axis, for disposing said used medical syringe into said container storage space, whereby,
(a) when a used pen needle is positioned in said pen needle receiving means and said manually rotatable means is rotated a pre-selected amount about said rotational axis from said position of said initial contacting so that said cam follower means progressively contacts said cam means to move said ejector means along said ejector axis to force said used pen needle axially out of said pen needle receiving means into said container storage space, and
(b) when a used medical syringe is received by said means included in said rotatable means for receiving a used medical syringe and said rotatable means is rotated said pre-selected rotation about said rotational axis, said used medical syringe will be disposed into said container storage space.

10. The sharps container of claim 9 wherein the container also facilitates the insertion, for safe disposal, and the safe storage of used medical syringes (260) into said container.

11. The sharps container of claim 10 further **characterized by** rotatable means comprising:
(i) an elongated cylindrically shaped member supported on opposite ends thereof on said housing means and disposed in an elongated opening (220) in said housing sized to receive said elongated cylindrically shaped member, and
(ii) at least one elongated recess in said elongated cylindrically shaped member sized to receive at least one used medical syringe (260).

12. The sharps container of claim 11 including said used pen needle ejector assembly being mounted on said elongated cylindrically shaped member with said ejector axis being oriented at an angle to said rotational axis.

13. The sharps container of claim 12 wherein
(i) said elongated cylindrically shaped member has two of said elongated recesses disposed longitudinally on opposite sides of said rotational axis, and
(ii) said used pen needle ejector assembly is mounted, as aforesaid, so that said ejector axis is normal to said rotational axis.

14. The sharps container of claim 13 further **characterized by** said elongated means of said ejector assembly including two, axially aligned and axially spaced apart tubular means connected to said elongated cylindrically shaped member and having bore means for receiving said ejector member.

15. The sharps container of claim 14 wherein a first of said tubular means has recessed means at an end thereof sized to receive a used pen needle.

16. The sharps container of claim 13 wherein said cylindrically shaped member has a longitudinally extending central core portion including a central means for supporting said elongated ejector member for movement, relative to said cylindrically shaped member, along said ejector axis.

17. The sharps container of claim 16 wherein said cylindrically shaped member includes first and second axially aligned and axially spaced apart tubular means axially aligned with and on opposite sides of said central means, said tubular means having bore means for receiving said elongated ejector member, and said first of said tubular means having means for receiving a used pen needle.

18. The sharps container of claim 17 wherein said elongated ejector member is initially positioned, for relative axial motion along said ejector axis relative to said aligned central means and said first and second tubular means, so that
(i) said cam follower means is positioned a pre-selected distance beyond said second of said tubular means, and
(ii) said second end of said ejector member is positioned adjacent to said means for receiving a used pen needle.

19. The sharps container of claim 18 wherein said second end of said elongated ejector member includes means for accommodating an axially extending used needle attached to a pen needle positioned in said used needle receiving means.

20. The sharps container of claim 11 wherein said rotatable cylindrically shaped member is **characterized** so that said at least one elongated recess is parallel to said rotational axis and is adapted, when in a first angular orientation relative to said housing means, to receive a used medical syringe and is adapted, when in a second angular position relative to said housing means, to dispose of said used medical syringe into said container.

21. The sharps container of claim 13 wherein said ejector axis is positioned intermediate said two longitudinally disposed recesses.

22. The sharps container of claim 18 wherein said second end of said elongated ejector member comprises two axially extending legs laterally spaced-apart a pre-selected distance for permitting the unobstructed positioning therebetween of a used needle of a pen needle received by said used pen needle receiving means.

23. The sharps container of claim 18 including means on said elongated ejector member for limiting the axial displacement of said cam follower means beyond said preselected axial distance along said ejector axis.

24. The sharps container of claim 23 wherein said means for limiting axial displacement of said cam follower means includes
(i) a pair of spaced apart legs integral with said elongated ejector member, and
(ii) shoulder means on said housing means.

25. The sharps container of claim 1 wherein the container further facilitates
(i) the safe transportation of a large number of unused pen needle assemblies,
(ii) the safe dispensing of said unused pen needle assemblies from said container, wherein said housing means is sized to hold, in at least a portion thereof, a plurality of unused pen needle assemblies, said housing having an opening for facilitating the withdrawal of unused pen needle assemblies.

## Patentansprüche

1. Kanülensammelbehälter zur Aufbewahrung von gebrauchten Penkanülen, umfassend:
ein Gehäuse (10) mit einem Behälteraufbewahrungsraum (11), der dimensioniert ist, um die Aufbewahrung einer Vielzahl von gebrauchten Penkanülen zu erleichtern, wobei der Behälter eine Öffnung (13) in dem Gehäuse enthält, die dimensioniert ist, um die axiale Einführung einer gebrauchten Penkanüle durch diese hindurch zuzulassen; und
innerhalb des Gehäuses eine Einrichtung zum Aufnehmen und Auswerfen gebrauchter Penkanülen; wobei die Einrichtung zum Aufnehmen und Auswerfen gebrauchter Penkanülen umfasst:
eine rotierbare Einrichtung (15, 16), die mit dem Gehäuse zur relativen Drehung um eine Achse (RA) mit diesem verbunden ist; eine Auswerfereinheit (30), die mit der rotierbaren Einrichtung verbunden ist, um um diese Achse gedreht zu werden; und eine Mitnehmereinrichtung (212) innerhalb des Gehäuses, wobei die Auswerfereinheit umfasst:
ein längliches, rohrförmiges Element (31) mit Ausnehmungsmitteln (33) an einem ersten Ende davon, so dimensioniert, um eine gebrauchte Penkanüle (50) aufzunehmen;
ein Auswerferelement (40', 240), das mit dem länglichen, rohrförmigen Element zur relativen axialen Bewegung darin entlang einer Auswerferachse verbunden ist und mit
(A) einer Kurvenstößeleinrichtung (40B, 240C) an einem Ende davon, die zu Anfang in einem vorgewählten, axialen Abstand entlang der Auswerferachse über ein zweites Ende des länglichen, rohrförmigen Elementes hinaus angeordnet ist, und
(B) einem zweiten Ende, das zu Anfang angrenzend an die Ausnehmungsmittel (33) angeordnet ist, um die gebrauchte Penkanüle aufzunehmen, und wobei die Mitnehmereinrichtung für eine Anfangsberührung der Kurvenstößeleinrichtung positioniert wird, wenn das rotierbare Element und die Auswerfereinheit um die Drehachse gedreht werden, wobei die Mitnehmereinrichtung das Auswerferelement (240) axial gegenüber dem länglichen, rohrförmigen Element zu dem ersten Ende hin bewegt, so dass ein zweites Ende des Auswerferelementes (40', 240) eine Penkanülen-Auswerfbewegung bewirkt, wodurch das Auswerferelement, wenn eine Penkanüle (50) in den Ausnehmungsmitteln (33) angeordnet ist und die rotierbare Einrichtung (15, 16) um die Achse (RA) genügend gedreht wird, so dass die Kurvenstößeleinrichtung die Mitnehmereinrichtung berührt, die Penkanüle in axialer Richtung aus den Ausnehmungsmitteln heraus in den Behälteraufbewahrungsraum (11) drücken wird,
**dadurch gekennzeichnet, dass** die Auswerfereinheit (30) wahlweise um die Achse (RA) ausgerichtet wird durch Drehung der rotierbaren Einrichtung zwischen
(i) einer Anfangsstellung, in der die Ausnehmungsmittel der Öffnung (13) benachbart und mit dieser in einer Linie sind, um die axiale Einführung einer gebrauchten Penkanüle in die Ausnehmungsmittel zu erleichtern, und
(ii) einer zweiten Stellung, an der die Kurvenstößeleinrichtung mit der Mitnehmereinrichtung in Eingriff kommt, woraus sich ergibt, dass eine zusätzliche Drehung der Auswerfereinheit um die Achse (RA) das Auswerfen einer gebrauchten Penkanüle aus den Ausnehmungsmitteln in das Gehäuse zur sicheren Aufbewahrung darin bewirkt.

2. Kanülensammelbehälter nach Anspruch 1, bei dem
die Auswerfereinheit (30) ein Element umfasst mit
(i) einer länglichen Bohrungseinrichtung vorgewählter axialer Länge und einem ersten und einem zweiten Ende, wobei das erste Ende der Bohrungseinrichtung so dimensioniert ist, um den vorgewählten Außendurchmesser einer Penkanüle aufzunehmen;
(ii) wobei die Bohrungseinrichtung Ansatzmittel (231) aufweist, die an das erste Ende angrenzen, und
(iii) einer länglichen Auswerfereinrichtung, die innerhalb der Bohrungseinrichtung mit Verriegelungsmitteln (241, 242) an einem Ende davon zum Eingriff mit den Ansatzmitteln und mit der Kurvenstößeleinrichtung an einem zweiten Ende davon angeordnet ist, wobei die Auswerfereinrichtung axial so dimensioniert ist, dass sich die Kurvenstößeleinrichtung über das zweite Ende der Bohrungseinrichtung hinaus erstreckt, wenn sich die Verriegelungsmittel mit den Ansatzmittel in Eingriff befinden,
wobei die Mitnehmereinrichtung die Verriegelungsmittel (241) der Auswerfereinrichtung axial von den Ansatzmitteln weg, hin zu dem ersten Ende der Bohrungseinrichtung bewegt, um eine Auswerfbewegung zu bewirken.

3. Kanülensammelbehälter nach Anspruch 2, bei dem
die Auswerfereinheit ein Rohrmittel vorgewählter axialer Länge mit einem ersten und einem zweiten Ende sowie einer Innenbohrung aufweist, in der die Auswerfereinrichtung bewegbar angeordnet ist..

4. Kanülensammelbehälter nach Anspruch 3, bei dem
die Innenbohrung einen an das erste Ende des Rohrmittels angrenzenden Ansatz enthält und der Teil der Innenbohrung zwischen dem ersten Ende des Rohrmittels und den Ansatzmitteln so dimensioniert ist, um den vorgewählten Außendurchmesser einer Penkanüle dicht aufzunehmen.

5. Kanülensammelbehälter nach Anspruch 4, bei dem
der Teil der Innenbohrung eine sich radial erstreckende Einrichtung zum Greifen des vorgewählten Außendurchmessers einer Penkanüle enthält.

6. Kanülensammelbehälter nach Anspruch 4, umfassend
eine Öffnung in dem Gehäuse, die so dimensioniert ist, um die axiale Einführung einer Penkanüle durch diese hindurch zu erlauben, wobei die Einrichtung zum Aufnehmen und Auswerfen gebrauchter Penkanülen gegenüber der Öffnung so ausgerichtet ist, dass das erste Ende der Bohrungseinrichtung in enger Nähe zu der Öffnung gedreht werden kann, um die Einführung einer gebrauchten Penkanüle darin zu erleichtern.

7. Kanülensammelbehälter nach Anspruch 4, bei dem
die Mitnehmereinrichtung mit dem Gehäuse einstückig ist.

8. Kanülensammelbehälter nach Anspruch 1, bei dem
die Ausnehmungsmittel des länglichen Elementes sich radial erstreckende Mittel enthalten.

9. Kanülensammelbehälter nach Anspruch 1, des Weiteren umfassend:
eine Einrichtung zum Aufnehmen und Entsorgen von gebrauchten medizinischen Spritzen weist Mittel auf, die in der rotierbaren Einrichtung enthalten sind zum
(i) Aufnehmen einer gebrauchten medizinischen Spritze (260), und
(ii) zum Folgenlassen einer vorgewählten Drehung der rotierbaren Einrichtung um die Drehachse, um die gebrauchte medizinische Spritze in den Behälteraufbewahrungsraum zu entsorgen, wodurch
wenn (a) eine gebrauchte Penkanüle in der Penkanülenaufnahmeeinrichtung angeordnet ist und die manuell rotierbare Einrichtung eine vorgewählte Größe um die Drehachse von der Position der anfänglichen Kontaktherstellung gedreht wird, so dass die Kurvenstößeleinrichtung fortschreitend mit der Mitnehmereinrichtung in Kontakt kommt zum Bewegen der Auswerfereinrichtung entlang der Auswerferachse, um die gebrauchte Penkanüle in axialer Richtung aus der Penkanülenaufnahmeeinrichtung in den Behälteraufbewahrungsraum zu drücken, und
wenn (b) eine gebrauchte medizinische Spritze durch die in der rotierbaren Einrichtung enthaltenen Einrichtung zum Aufnehmen einer gebrauchten medizinischen Spritze aufgenommen ist und die rotierbare Einrichtung die vorgewählte Drehung um die Drehachse gedreht wird, die gebrauchte medizinische Spritze in den Behälteraufbewahrungsraum entsorgt werden wird.

10. Kanülensammelbehälter nach Anspruch 9, wobei der Behälter außerdem die Einführung zur sicheren Entsorgung und die sichere Aufbewahrung von gebrauchten medizinischen Spritzen (260) in den Behälter erleichtert.

11. Kanülensammelbehälter nach Anspruch 10, des Weiteren **gekennzeichnet durch** eine rotierbare Einrichtung, umfassend:
(i) ein längliches, zylindrisch geformtes Element, das an gegenüber liegenden Enden davon an der Gehäuseeinrichtung gehalten wird und in einer länglichen Öffnung (220) in dem Gehäuse angeordnet ist, so dimensioniert, um das längliche, zylindrisch geformte Element aufzunehmen, und
(ii) zumindest eine längliche Ausnehmung in dem länglichen, zylindrisch geformten Element, so dimensioniert, um zumindest eine gebrauchte medizinische Spritze (260) aufzunehmen.

12. Kanülensammelbehälter nach Anspruch 11, umfassend, dass die Auswerfereinheit für gebrauchte Penkanülen an dem länglichen, zylindrisch geformten Element angebracht ist, wobei die Auswerferachse in einem Winkel zu der Drehachse ausgerichtet ist.

13. Kanülensammelbehälter nach Anspruch 12, bei dem
(i) das längliche, zylindrisch geformte Element zwei der länglichen Ausnehmungen aufweist, die in Längsrichtung an gegenüber liegenden Seiten der Drehachse angeordnet sind, und
(ii) die Auswerfereinheit für gebrauchte Penkanülen, wie oben erwähnt, so angebracht ist, dass die Auswerferachse senkrecht zu der Drehachse ist.

14. Kanülensammelbehälter nach Anspruch 13, des Weiteren **dadurch gekennzeichnet, dass** das längliche Mittel der Auswerfereinheit zwei axial ausgerichtete und axial beabstandete rohrförmige Mittel enthält, die mit dem länglichen, zylindrisch geformten Element verbunden sind und Bohrungseinrichtungen zum Aufnehmen des Auswerferelements aufweisen.

15. Kanülensammelbehälter nach Anspruch 14, bei dem
eine erste der rohrförmigen Einrichtungen Ausnehmungsmittel an deren einem Ende aufweist, die so dimensioniert sind, um eine gebrauchte Penkanüle aufzunehmen.

16. Kanülensammelbehälter nach Anspruch 13, bei dem
das zylindrisch geformte Element einen sich in Längsrichtung erstreckenden, mittleren Kernabschnitt mit einer mittleren Einrichtung zum Halten des länglichen Auswerferelements zur Bewegung relativ zu dem zylindrisch geformten Element entlang der Auswerferachse aufweist.

17. Kanülensammelbehälter nach Anspruch 16, bei dem
das zylindrisch geformte Element erste und zweite, axial ausgerichtete und axial beabstandete, rohrförmige Einrichtungen enthält, die axial mit und an gegenüber liegenden Seiten der mittleren Einrichtung ausgerichtet sind, wobei die rohrförmigen Einrichtungen Bohrungseinrichtungen zum Aufnehmen des länglichen Auswerferelements aufweisen, und die erste der rohrförmigen Einrichtungen Mittel zum Aufnehmen einer gebrauchten Penkanüle aufweist.

18. Kanülensammelbehälter nach Anspruch 17, bei dem
das längliche Auswerferelement zu Anfang zur relativen axialen Bewegung entlang der Auswerferachse relativ zu der ausgerichteten mittleren Einrichtung und der ersten und der zweiten rohrförmigen Einrichtung positioniert wird, so dass
(i) die Kurvenstößeleinrichtung in einem vorgewählten Abstand über die zweite der rohrförmigen Einrichtungen hinaus positioniert ist, und
(ii) das zweite Ende des Auswerferelements angrenzend an die Einrichtung zum Aufnehmen einer gebrauchten Penkanüle positioniert ist.

19. Kanülensammelbehälter nach Anspruch 18, bei dem
das zweite Ende des länglichen Auswerferelements eine Einrichtung zur Aufnahme einer sich axial erstreckenden, gebrauchten Kanüle umfasst, die an einer in der Aufnahmeeinrichtung für gebrauchte Kanülen positionierten Penkanüle befestigt ist.

20. Kanülensammelbehälter nach Anspruch 11, bei dem
das drehbare, zylindrisch geformte Element so **gekennzeichnet** ist, dass die zumindest eine längliche Ausnehmung parallel zu der Drehachse ist und bei einer ersten Winkelorientierung relativ zu der Gehäuseeinrichtung angepasst wird, um eine gebrauchte medizinische Spritze aufzunehmen, und bei einer zweiten Winkelposition relativ zu der Gehäuseeinrichtung angepasst wird, um die gebrauchte medizinische Spritze in den Behälter zu entsorgen.

21. Kanülensammelbehälter nach Anspruch 13, bei dem
die Auswerferachse zwischen den zwei in Längsrichtung angeordneten Ausnehmungen positioniert ist.

22. Kanülensammelbehälter nach Anspruch 18, bei dem
das zweite Ende des länglichen Auswerferelements zwei, sich axial erstreckende Schenkel aufweist, die seitlich in einem vorgewählten Abstand beabstandet sind, um dazwischen das unbehinderte Positionieren einer gebrauchten Kanüle einer durch die Aufnahmeinrichtung für gebrauchte Penkanülen aufgenommenen Penkanüle zu erlauben.

23. Kanülensammelbehälter nach Anspruch 18, umfassend
eine Einrichtung an dem länglichen Auswerferelement zum Begrenzen der axialen Verschiebung der Kurvenstößeleinrichtung über den vorgewählten axialen Abstand hinaus entlang der Auswerferachse.

24. Kanülensammelbehälter nach Anspruch 23, bei dem
die Einrichtung zum Begrenzen einer axialen Verschiebung der Kurvenstößeleinrichtung umfasst
(i) ein Paar beabstandeter Schenkel, die mit dem länglichen Auswerferelement einstückig sind, und
(ii) Ansatzmittel an der Gehäuseeinrichtung.

25. Kanülensammelbehälter nach Anspruch 1, wobei der Behälter des Weiteren
(i) den sicheren Transport einer großen Anzahl von ungebrauchten Penkanüleneinheiten,
(ii) die sichere Abgabe der ungebrauchten Penkanüleneinheiten aus dem Behälter erleichtert, wobei die Gehäuseeinrichtung so dimensioniert ist, um zumindest in einem Teil davon eine Vielzahl von ungebrauchten Penkanüleneinheiten aufzunehmen, wobei das Gehäuse eine Öffnung aufweist, um die Entnahme von ungebrauchten Penkanüleneinheiten zu erleichtern.

## Revendications

1. Conteneur d'objets pointus pour le stockage d'aiguilles de stylo usagées comprenant :
un boîtier (10) ayant un espace de stockage de conteneur (11) dimensionné pour faciliter le stockage d'une pluralité d'aiguilles de stylo usagées, le conteneur comprenant une ouverture (13) dans ledit boîtier dimensionné pour admettre l'insertion axiale d'une aiguille de stylo usagée à travers celle-ci, et des moyens de réception et d'éjection d'aiguille de stylo usagée, les moyens de réception et d'éjection d'aiguille usagée comprenant :
des moyens rotatifs (15, 16) reliés audit boîtier pour une rotation par rapport audit boîtier autour d'un axe (RA), un ensemble éjecteur (30) relié auxdits moyens rotatifs pour pouvoir être tourné autour dudit axe ; et des moyens formant came (212) dans ledit boîtier, l'ensemble éjecteur comprenant :
un élément tubulaire allongé (31) ayant des moyens évidés (33) à une première extrémité de celui-ci, dimensionné pour recevoir une aiguille de stylo usagée (50) ;
un élément éjecteur (40', 240) relié audit élément tubulaire allongé pour un déplacement axial relatif dans celui-ci le long d'un axe d'éjecteur, et ayant
(A) des moyens formant suiveur de came (40B, 240C) à une première extrémité de celui-ci, positionnés initialement à une distance axial présélectionnée le long dudit axe d'éjecteur au-delà d'une seconde extrémité dudit élément tubulaire allongé, et
(B) une seconde extrémité positionnée initialement adjacente auxdits moyens évidés (33) pour recevoir ladite aiguille de stylo usagée et où lesdits moyens formant suiveur de came étant positionnés pour une mise encontact initiale desdits moyens formant suiveur de came lorsque ledit élément rotatif et ledit ensemble éjecteur sont tournés autour dudit axe de rotation, dans lequel lesdits moyens formant came déplacent ledit élément éjecteur (240) axialement par rapport audit élément tubulaire allongé vers ladite première extrémité, de sorte qu'une seconde extrémité dudit élément éjecteur (40', 240) fournit un mouvement d'éjection d'aiguille de stylo, de sorte que, lorsqu'une aiguille de stylo (50) est positionnée dans lesdits moyens évidés (33), et lesdits moyens rotatifs (15, 16) sont tournés autour dudit axe (RA) suffisamment pour que lesdits moyens formant suiveur de came viennent en contact avec lesdits moyens formant came, ledit élément éjecteur va pousser ladite aiguille de stylo axialement en dehors desdites moyens évidés jusque dans ledit espace de stockage de conteneur (11),
**caractérisé en ce que** ledit ensemble éjecteur (30) est orienté sélectivement autour dudit axe (RA) par une rotation desdits moyens rotatifs entre
(i) une position initiale dans laquelle lesdits moyens évidés sont adjacents à ladite ouverture (13), et alignés avec celle-ci, pour faciliter l'insertion axiale d'une aiguille de stylo usagée dans lesdits moyens évidés, et
ii) une seconde position dans laquelle lesdits moyens formant suiveur de came viennent en contact avec lesdits moyens formant came, à la suite de quoi une rotation supplémentaire dudit ensemble éjecteur autour dudit axe (RA) provoque l'éjection d'une aiguille de stylo usagée depuis lesdits moyens évidés jusque dans ledit boîtier, pour un stockage sûr dans celui-ci.

2. Conteneur d'objets pointus selon la revendication 1, dans lequel ledit ensemble éjecteur (30) comprend un élément ayant
i) des moyens formant alésage allongé d'une longueur axiale présélectionnée, et ayant des première et seconde extrémités, ladite première extrémité desdits moyens formant alésage étant dimensionnée pour recevoir ledit diamètre extérieur présélectionné d'une aiguille de stylo,
(ii) lesdits moyens formant alésage ayant des moyens d'épaulement (231) adjacents à ladite première extrémité, et
(iii) des moyens éjecteurs allongés positionnés dans lesdits moyens formant alésage, avec des moyens de verrouillage (241, 242) à une première extrémité de ceux-ci pour une mise en prise avec lesdits moyens d'épaulement, et des moyens formant suiveur de came à une seconde extrémité de ceux-ci, lesdits moyens éjecteurs étant dimensionnés axialement de sorte que lesdits moyens formant suiveur de came s'étendent au-delà de ladite seconde extrémité desdits moyens formant alésage lorsque les moyens de verrouillage sont en prise avec lesdits moyens d'épaulement,
dans lequel lesdits moyens formant came déplacent lesdits moyens de verrouillage (241) desdits moyens éjecteurs axialement loin desdits moyens d'épaulement vers ladite première extrémité desdits moyens formant alésage, pour fournir un mouvement d'éjection.

3. Conteneur d'objets pointus selon la revendication 2, dans lequel ledit ensemble éjecteur comprend des moyens tubulaires d'une longueur axiale présélectionnée ayant des première et seconde extrémités, et un alésage interne à l'intérieur duquel lesdits moyens éjecteurs sont positionnés de manière mobile.

4. Conteneur d'objets pointus selon la revendication 3, dans lequel ledit alésage interne comprend un épaulement adjacent à ladite première extrémité desdits moyens tubulaires, et la partie dudit alésage interne située entre ladite première extrémité desdits moyens tubulaires et lesdits moyens d'épaulement est dimensionnée pour recevoir de manière serrée ledit diamètre extérieur présélectionné d'une aiguille de stylo.

5. Conteneur d'objets pointus selon la revendication 4, dans lequel ladite partie dudit alésage interne comprend des moyens s'étendant radialement pour saisir ledit diamètre extérieur présélectionné d'une aiguille de stylo.

6. Conteneur d'objets pointus selon la revendication 4, comprenant une ouverture dans ledit boîtier, dimensionnée pour permettre l'insertion axiale d'une aiguille de stylo à travers celle-ci, et lesdits moyens de réception et d'éjection d'aiguille de stylo usagée étant orientés par rapport à ladite ouverture de telle sorte que ladite première extrémité desdits moyens formant alésage peut être tournée à proximité étroite de ladite ouverture pour faciliter l'insertion d'un aiguille de stylo usagée dans celle-ci.

7. Conteneur d'objets pointus selon la revendication 4, dans laquelle lesdits moyens formant came sont en un seul bloc avec ledit boîtier.

8. Conteneur d'objets pointus selon la revendication 1, dans lequel lesdits moyens évidés dudit élément allongé comprennent des moyens s'étendant radialement.

9. Conteneur d'objets pointus selon la revendication 1, comprenant en outre :
des moyens de réception et de mise au rebut de seringue médicale usagée comprenant des moyens inclus dans lesdits moyens rotatifs
(ii) pour recevoir une seringue médicale usagée (260), et
(ii) à la suite d'une rotation présélectionnée desdits moyens rotatifs autour dudit axe de rotation, pour mettre au rebut ladite seringue médicale usagée dans ledit espace de stockage de conteneur, de telle sorte que,
(a) lorsqu'une aiguille de stylo usagée est positionnée dans lesdits moyens de réception d'aiguille de stylo, et lesdits moyens rotatifs manuellement sont tournés d'une quantité présélectionnée autour dudit axe de rotation depuis ladite position de ladite mise en contact initiale de telle sorte que les moyens formant suiveur de came viennent en contact progressivement avec lesdits moyens formant came pour déplacer lesdits moyens éjecteurs le long dudit axe d'éjecteur afin de pousser ladite aiguille de stylo usagée axialement à l'extérieur desdits moyens de réception d'aiguille de stylo jusque dans ledit espace de stockage de conteneur, et
(b) lorsqu'une seringue médicale usagée est reçue par lesdits moyens inclus dans lesdits moyens rotatifs pour recevoir une seringue médicale usagée, et lesdits moyens rotatifs sont tournés de ladite rotation présélectionnée autour dudit axe de rotation, ladite seringue médicale usagée va être mise au rebut dans ledit espace de stockage de conteneur.

10. Conteneur d'objets pointus selon la revendication 9, dans lequel le conteneur facilite également l'insertion, pour une mise au rebut sûre, et le stockage sûr de seringues médicales usagées (260), jusque dans ledit conteneur.

11. Conteneur d'objets pointus selon la revendication 10, **caractérisé en outre par** des moyens rotatifs, comprenant :
(i) un élément allongé mis en forme de manière cylindrique supporté sur des extrémités opposées de celui-ci sur lesdits moyens de boîtier, et disposé dans une ouverture allongée (120) située dans ledit boîtier, dimensionnée pour recevoir ledit élément allongé mis en forme de manière cylindrique, et
(ii) au moins un évidement allongé dans ledit élément allongé mis en forme de manière cylindrique, dimensionné pour recevoir au moins une seringue médicale usagée (260).

12. Conteneur d'objets pointus selon la revendication 11, comprenant ledit ensemble éjecteur d'aiguille de stylo usagée étant monté sur ledit élément allongé mis en forme de manière cylindrique en ayant ledit axe d'éjecteur orienté sur un angle par rapport audit axe de rotation.

13. Conteneur d'objets pointus selon la revendication 12, dans lequel
(i) ledit élément allongé mis en forme de manière cylindrique a deux desdits évidements allongés disposés longitudinalement sur des côtés opposés dudit axe de rotation, et
(ii) ledit ensemble éjecteur d'aiguille de stylo usagée est monté, comme mentionné précédemment, de sorte que ledit axe d'éjecteur est perpendiculaire audit axe de rotation.

14. Conteneur d'objets pointus selon la revendication 13, **caractérisé en outre par** lesdits moyens allongés dudit ensemble éjecteur comprenant deux moyens tubulaires espacés et alignés axialement, reliés audit élément allongé mis en forme de manière cylindrique, et ayant des moyens formant alésage pour recevoir ledit élément éjecteur.

15. Conteneur d'objets pointus selon la revendication 14, dans lequel un premier desdits moyens tubulaires a des moyens évidés à une extrémité de celui-ci, dimensionnés pour recevoir une aiguille de stylo usagée.

16. Conteneur d'objets pointus selon la revendication 13, dans lequel ledit élément mis en forme de manière cylindrique a une partie de coeur centrale s'étendant longitudinalement comprenant des moyens centraux pour supporter ledit élément éjecteur allongé pour un déplacement, par rapport audit élément mis en forme de manière cylindrique, le long dudit axe d'éjecteur.

17. Conteneur d'objets pointus selon la revendication 16, dans lequel ledit élément mis en forme de manière cylindrique comprend des premiers et seconds moyens tubulaires espacés et alignés axialement, et alignés axialement avec et sur des côtés opposés desdits moyens centraux, lesdits moyens tubulaires ayant des moyens formant alésage pour recevoir ledit élément éjecteur allongé, et lesdits premiers desdits moyens tubulaires ayant des moyens pour recevoir une aiguille de stylo usagée.

18. Conteneur d'objets pointus selon la revendication 17, dans lequel ledit élément éjecteur allongé est positionné initialement pour un mouvement axial relatif le long dudit axe d'éjecteur par rapport auxdits moyens centraux alignés, et auxdits premiers et seconds moyens tubulaires, de sorte que
(i) lesdits moyens formant suiveur de came sont positionnés à une distance présélectionnée au-delà desdits seconds moyens tubulaires, et
(ii) ladite seconde extrémité dudit élément injecteur est positionnée adjacent auxdits moyens pour recevoir une aiguille de stylo usagée.

19. Conteneur d'objets pointus selon la revendication 18, dans lequel ladite seconde extrémité dudit élément éjecteur allongé comprend des moyens pour recevoir une aiguille usagée s'étendant axialement fixés sur une aiguille de stylo positionnée dans lesdits moyens de réception d'aiguille usagée.

20. Conteneur d'objets pointus selon la revendication 11, dans lequel ledit élément mis en forme de manière cylindrique rotatif est **caractérisé** de sorte que ledit au moins un évidement allongé est parallèle audit axe de rotation et est adapté, dans une première orientation angulaire par rapport auxdits moyens formant boîtier, pour recevoir une seringue médicale allongée, et est adapté, dans une seconde position angulaire par rapport auxdits moyens formant boîtier, pour mettre au rebut ladite seringue médical usagée dans ledit conteneur.

21. Conteneur d'objets pointus selon la revendication 13, dans lequel ledit axe d'éjecteur est positionné entre lesdits deux évidements disposés longitudinalement.

22. Conteneur d'objets pointus selon la revendication 18, dans lequel ladite seconde extrémité dudit élément éjecteur comprend deux pattes s'étendant axialement et espacées latéralement d'une distance présélectionnée, pour permettre le positionnement non obstrué entre celles-ci d'une aiguille usagée d'une aiguille de stylo reçue par lesdits moyens de réception d'aiguille de stylo usagée.

23. Conteneur d'objets pointus selon la revendication 18, comprenant des moyens sur ledit élément éjecteur allongé pour limiter le déplacement axial desdits moyens formant suiveur de came au-delà de ladite distance axiale présélectionnée le long dudit axe d'éjecteur.

24. Conteneur d'objets pointus selon la revendication 23, dans lequel lesdits moyens pour limiter un déplacement axial desdits moyens formant suiveur de came comprennent
(i) une paire de pattes espacées en un seul bloc avec ledit élément éjecteur allongé, et
(ii) des moyens d'épaulement sur lesdits moyens formant boîtier.

25. Conteneur d'objets pointus selon la revendication 1, dans lequel le conteneur facilite en outre
(i) le transport sûr d'un grand nombre d'ensembles d'aiguille de stylo non usagée, et
(ii) la distribution sûre desdits ensembles d'aiguille de stylo non usagée depuis ledit conteneur, dans lequel lesdits moyens formant boîtier sont dimensionnés pour contenir, dans au moins une partie de ceux-ci, une pluralité d'ensembles d'aiguille de stylo non usagée, ledit boîtier ayant une ouverture pour faciliter le retrait d'ensembles d'aiguille de stylo non usagée.
